**Europäisches Patentamt**

(19) **European Patent Office**

**Office européen des brevets**

(11) Publication number : **0 424 042 B1**

(12) **EUROPEAN PATENT SPECIFICATION**

(45) Date of publication of patent specification :
02.03.94 Bulletin 94/09

(51) Int. Cl.⁵ : **A61K 9/16,** A61K 9/06,
A61K 9/52

(21) Application number : **90311207.6**

(22) Date of filing : **12.10.90**

(54) **Non-solid ophthalmic composition and process for preparing the said composition.**

(30) Priority : **17.10.89 FR 8913533**

(43) Date of publication of application :
**24.04.91 Bulletin 91/17**

(45) Publication of the grant of the patent :
**02.03.94 Bulletin 94/09**

(84) Designated Contracting States :
**AT BE CH DE DK ES FR GB IT LI LU NL SE**

(56) References cited :
**EP-A- 0 227 494**
**WO-A-89/06964**

(73) Proprietor : **LABORATOIRES MERCK, SHARP
& DOHME-CHIBRET
3, Avenue Hoche
F-75008 Paris (FR)**

(72) Inventor : **Rozier, Annouk
23 Bd Lafayette
F-63000 Clermont-Ferrand (FR)**

(74) Representative : **Cole, William Gwyn et al
European Patent Department Merck & Co.,
Inc. Terlings Park Eastwick Road
Harlow Essex CM20 2QR (GB)**

Jouve, 18, rue Saint-Denis, 75001 PARIS

## Description

The present invention relates to a non-solid ophthalmic composition of the delayed-acting type.

Most known ophthalmic compositions do not enable a delay effect to be obtained. In practice, the watersoluble active principles administered in an aqueous matrix are rapidly eluted by the continual flow of lachrymal fluid. As regards solutions, these are rapidly removed from the area of absorption by lachrymal drainage. Moreover, the use of solid forms such as inserts can cause problems of pain or discomfort from the user's standpoint.

WO-A-89/06964 and EP-A-0227494 both describe ophthalmic compositions which are administered in liquid form and subsequently undergo gellation in situ in order to deliver an ophthalmic medicament to the active site in a sustained manner. In WO-A-89/06964, the in situ gellation is brought about by incorporating into the composition an aqueous suspension of a lightly crosslinked polymer prepared by polymerizing one or more carboxyl-containing monoethylenically unsaturated monomers, e.g. acrylic acid, and a crosslinking agent such as divinyl glycol. In EP-A-0227494, meanwhile, the in situ gellation is achieved by incorporating into the composition an aqueous solution of a polysaccharide, in particular gellan gum, which possesses the property of undergoing liquid-gel phase transition under the effect of the increase in ionic strength encountered by the composition upon contacting the lachrymal fluid.

The object of the present invention is to develop a non-solid composition, in particular a fluid composition, which nevertheless makes it possible to obtain a delay effect which can, if desired, be varied according to the activity sought.

More specifically, the present invention relates to a non-solid ophthalmic composition, characterized in that it contains a suspension of polymeric microparticles in a non-solid medium compatible with ophthalmic application, said polymeric microparticles being formed from a polycarboxylic polymer or a polycationic polymer and having a pH of dissolution lying between pH 4.5 and pH 8.5, the pH of the composition before application being predetermined such that it is further away from pH 7.4 than the dissolution pH of the polymer which constitutes the microparticles, thereby not permitting dissolution of the microparticles before application.

In order for the active principle to be able to act, the microparticles have to dissolve, that is to say the pH of the composition has to reach the pH of dissolution through the neutralizing action of the lachrymal fluid, which will tend to bring the pH back towards the normal pH of 7.4.

Moreover, it is preferable that the microparticles have no tendency to swell in an aqueous medium or under the influence of pH variations, since this phenomenon can lead to a porous structure from which the watersoluble active principle can be rapidly removed, leaving an empty matrix.

The phenomenon employed should preferably be the phenomenon of dissolution of the microparticle.

The polycarboxylic polymers, from which may be formed the microparticles employed in the composition according to the invention, are used, in particular, in enteric coatings. These polymers are insoluble in an aqueous medium at low pH and solubilize when the pH rises. The dissolution point varies for each polymer, for example it lies at pH 4.5 for cellulose acetate phthalate and at pH 7 for the copolymer of methacrylic acid and methyl methacrylate (Eudragit S100).

It is also possible to use polycationic polymers which are insoluble at pH values above 8 and soluble at lower pH values. The neutralizing properties of the lachrymal fluid tend to lower the pH and hence to dissolve microparticles.

The dissolution characteristics of the microparticles and the characteristics of the delay effect can be modified not only by using different polymers but also by modifying:
- the size,
- the active principle/polymer ratio,
- the physicochemical properties of the active principle, for example an active principle in base form will partially salify the carboxyl group of the polymer, and the salt obtained will be neutralized more quickly than the original polymer.

The size of the microparticles can vary within very wide limits, in particular from 1 to 200 μm and suitably from 1 to 70 μm. Preferred particle sizes are from 20 to 70 μm, especially from 30 to 60 μm.

In order that these microparticles remain in the conjunctival cul-de-sac for as long as possible, and in particular for a sufficient time to provide for neutralization and hence dissolution of the microparticles, the suspension medium will preferably be chosen from fluids, gels and phase-transition systems.

Thus, the suspension medium preferably consists of an aqueous solution of hydrophilic polymer, for example of polyvinyl alcohols, hydroxylated celluloses, polyvinyl pyrrolidone or poloxamers, as well as of derivatives of these compounds or alternatively of polysaccharides or natural or synthetic polysaccharide derivatives, and in particular phase-transition polymers. According to the invention, an extracellular anionic heteropolysaccharide produced by the bacterium Pseudomonas elodea, known by the name of gellan gum and marketed under the brand name Gelrite, will preferably be used.

It is also possible to modify the size and shape of the microparticles in order to increase their residence time in the conjunctival sac.

It is also possible to produce, according to the invention, a stepwise-release ophthalmic composition by incorporating an active principle also in the suspension medium. This active principle can be identical to or different from the active principle contained in the particles. The presence of this active principle in the medium makes it possible to have a high concentration of active principle immediately after introduction of the composition. It is possible, moreover, to provide for microparticles of a different nature and containing different active principles, if differing pharmacological activities are desired at different times.

It is possible to use a wide diversity of active principles. The latter may be chosen, in particular, from the following pharmaceutical compounds:

- antibacterial substances such as beta-lactam antibiotics, for example cefoxitin, n-formamidoylthienamycin and other thienamycin derivatives, tetracyclines, chloramphenicol, neomycin, carbenicillin, colistin, penicillin G, polymyxin B, vancomycin, cefazolin, cefaloridine, chibrorifamycin, gramicidin, bacitracin and sulphonomides;
aminoglycoside antibiotics such as gentamicin, kanamycin, amikacin, sisomicin and tobramycin;
nalidixic acid and its analogues such as norfloxacin and the antimicrobial combination of fluoroalanine/pentizidone, nitrofurazones and their analogues;

- antihistaminics and decongestants such as pyrilamine, chlorpheniramine, tetrahydrazoline, antazoline and their analogues;

- anti-inflammatories such as cortisone, hydrocortisone, hydrocortisone acetate, betamethasone, dexamethasone, dexamethasone sodium phosphate, prednisone, methylprednisolone, medrysone, fluorometholone, prednisolone, prednisolone sodium phosphate, triamcinolone, indomethacin, sulindac, its salts and corresponding sulphides, and their analogues;

- miotics and anticholinergics such as echothiopate, pilocarpine, physostigmine salicylate, diisopropyl fluorophosphate, epinephrine, dipivalylepinephrine, neostigmine, echothiopate iodide, demecarium bromide, carbamoylcholine chloride, methacholine, bethanechol and their analogues;

- Other drugs used in the treatment of eve conditions and lesions, such as:
antiglaucoma drugs, for example timolol and R-timolol and a combination of timolol or R-timolol with pilocarpine, and also many other adrenergic agonists and/or antagonists; epinephrines and a complex or epinephrine or prodrugs, and dipivefrin derivatives and hyperosmotic agents such as glycerol, mannitol and urea;
antiparasitic compounds and/or antiprotozoal compounds such as ivermectin, pyrimethamine, trisulphapyrimidine, clindamycin and corticosteroid preparations;
compounds having antiviral activity such as acyclovir, 5-iodo-2'-deoxyuridine (IDU), adenosine arabinoside (Ara-A), trifluorothymidine, and interferon and interferon-inducing agents such as polyI.polyC, carbonic anhydrase inhibitors such as acetazolamide, dicholorphenamide, 2-(p-hydroxyphenyl)thio-5-thiophenesulphonamide, 6-hydroxy-2-benzothiazolesulphonamide, 6-pivaloyloxy-2-benzothiazole-sulphonamide, MK297 and MK417;
antifungal agents such as amphotericin B, nystatin, flucytosine, natamycin and miconazole;
anaesthetic agents such as etidocaine, cocaine, benoxinate, dibucaine hydrochloride, dyclonine hydrochloride, naepaine, phenacaine hydrochloride, piperocaine, proparacaine hydrochloride, tetracaine hydrochloride, hexylcaine, bupivacaine, lidocaine, mepivacine and prilocaine;

- Ophthalmic diagnostic agents such as:
  a) those which are used for examining the retina, such as fluorescein sodium;
  b) those which are used for examining the conjunctiva, cornea and lachrymal apparatus, such as fluorescein and rose bengal; and
  c) those which are used for examining abnormal responses of the pupil, such as methacholine, cocaine, adrenaline, atropine, hydroxyamphetamine and pilocarpine;

- Ophthalmic agents used as surgical aids, such as alphachymotrypsin and hyaluronidase;
- Chelating agents such as ethylenediaminetetraacetic acid (EDTA) and deferoxamine;
- Immunosuppressants and antimetabolites such as methotrexate, cyclophosphamide, 6-mercaptopurine and azathioprine; and antibiotic/anti-inflammatory combinations such as the combination neomycin sulphate/dexamethasone sodium phosphate, and combinations concomitantly treating glaucoma, for example a timolol maleate/aceclidine combination.

The ophthalmic composition according to the invention must naturally fulfil the criteria for use in this field, in particular it should preferably be isotonic and be such that the polymeric microparticles contained therein have a pH of dissolution lying between 4.5 and 8.5. Thus, it may incorporate other components such as tonicity regulators and preservatives. Although buffer systems are customarily used, in the present case these systems may interfere with the dissolution and, if necessary, they may hence be omitted.

The present invention also relates to a process for preparing the ophthalmic compositions according to the present invention, characterized in that:

- the polymer forming the microparticles and the active principle are solubilized in a solvent,
- the solvent is then evaporated off and the polymer is formed into microparticles, and
- the microparticles obtained above are then mixed into a non-solid medium compatible with ophthalmic application and optionally containing the other ingredients, until a suspension is obtained.

The techniques employed for obtaining the microparticles depend on the polymer and the active principle. More often than not, since the polymers are soluble in organic solvents, a mixture of polymers and active principle in this solvent will be prepared and the solvent will then be evaporated off to form the particles. Thus, the polymer mass obtained may be fractionated and sieved in order to obtain microparticles, or alternatively the microparticles will be obtained by atomization.

The compositions according to the present invention have the advantage of being in liquid or gel form, and hence easier to administer than inserts, and of nevertheless possessing kinetics of release which provides for a presence of active principle for a fairly long time in the vicinity of the site of action.

Other features and advantages of the present invention will become apparent on reading the examples below.

## EXAMPLE 1 Formulation of an ophthalmic composition

- Microparticles        0.955 g
          32 < 0 < 56 μm
- Gelrite        0.60 g
- Mannitol        4.50 g
- Benzalkonium chloride        0.01 g
- Kollidon 12 PF        10.00 g
- Water        qs 100.0 g

## Composition of the microparticles

- Timolol base        0.250 g
- Eudragit L100        0.705 g
. mannitol is a tonicity regulator;
. benzalkonium chloride is a suitable preservative for ophthalmic use;
. timolol base is an active principle;
. Eudragit L100 is a copolymer of methacrylic acid and methyl methacrylate;
. Gelrite is marketed by Kelco; and
. Kollidon 12 PF is a low molecular weight polyvinylpyrrolidone marketed by BASF.

## Preparation of the ophthalmic composition

The timolol and the Eudragit are dissolved in acetone/ethanol/water mixture. The solution is applied in fine layers and the solvent then evaporated off. When the film has formed, it is finely ground in a mortar. The powder is then sieved and only the particles 32 to 56 μm in size are retained.

The powder is dispersed in the fluid dispersant medium containing the other ingredients, and the composition is brought to equilibrium for 24 hours with stirring. At this stage, 60% of the timolol is in the microparticles and 40% in the dispersant medium.

## EXAMPLE 2

## Kinetics of release of the active principle in vitro from an ophthalmic composition according to the invention

The release of the active principle takes place in three stages:

A first stage of approximately 30 minutes, characterized by a rapid release. This corresponds to the release of the active principle solubilized in the fluid dispersant medium.

A second stage of 30 to 60 minutes, with a slower diffusion. This corresponds to the dissolution time of the microparticles.

Then, a third stage where the diffusion profile of the active principle is identical to that observed for the active principle solubilized in the fluid dispersant medium, and which hence indicates that all the active principle is thereafter solubilized.

## EXAMPLE 3

**In vivo study of an ophthalmic composition according to the invention**

The ocular distribution of timolol in rabbits was assessed after topical administration of a composition according to the invention. Bilateral instillations of 30 μl of the test solutions were carried out in the conjunctival sac of living albino rabbits. Groups of three animals were sacrificed at 0.5, 1, 2 and 4 hours after administration and the timolol was assayed in the cornea, aqueous humor, iris and ciliary body.

The results appear in Table I.

For purposes of comparison, the table also shows ocular contents obtained with 0,25% of timolol maleate in an identical dispersant medium.

The concentration profile of active principle with the passage of time is completely modified with a composition according to the invention. This indicates a sustained release of the active principle.

TIMOLOL CONCENTRATIONS IN VARIOUS TISSUES OF THE EYE OF ALBINO RABBITS AFTER INSTILLATION
OF EUDRAGIT MICROPARTICLES CONTAINING 0.25% OF TIMOLOL ACCORDING TO THE INVENTION
(EXAMPLE 1) OR OF A CONTROL SOLUTION

| | 30 MIN | 60 MIN | 120 MIN | 240 MIN | Quantity of A.P. available $\mu g$ - hr/ml |
|---|---|---|---|---|---|
| Control Solution | | | | | |
| Aq. H. | 2.02 ± 0.26 | 2.79 ± 0.66 | 1.19 ± 0.61 | 0.12 ± 0.06 | 5.0 ± 1.6 |
| Iris + ciliary B | 2.71 ± 0.64 | 3.03 ± 0.84 | 2.27 ± 1.36(5) | 1.67 ± 0.51(2)* | 8.7 ± 3.5 |
| Cornea | 26.30 ± 5.12 | 21.20 ± 5.34 | 7.62 ± 3.18 | 1.28 ± 0.62 | 41.8 ± 2.9 |
| Composition according to the invention (Example 1) | | | | | |
| Aq. H. | 0.74 ± 0.10 | 0.76 ± 0.30 | 1.05 ± 0.39 | 0.11 ± 0.03 | 2.6 ± 0.9 |
| Iris + ciliary B | 1.90 ± 0.79 | 1.69 ± 0.56 | 3.31 ± 1.37(5) | 0.76 ± 0.05(2)* | 7.9 ± 2.9 |
| Cornea | 13.09 ± 3.16(5) | 10.99 ± 4.14 | 9.89 ± 3.52 | 1.41 ± 0.76 | 31.0 ± 10.7 |

The results are expressed in $\mu g/ml$ or $\mu g/g$ and represent
the means and standard deviation for 6 eyes.

EP 0 424 042 B1

## Claims

**Claims for the following Contracting States : AT, BE, CH, DE, DK, FR, GB, IT, LI, LU, NL, SE**

1. A non-solid ophthalmic composition, characterized in that it contains a suspension of polymeric microparticles in a non-solid medium compatible with ophthalmic application, said polymeric microparticles being formed from a polycarboxylic polymer or a polycationic polymer and having a pH of dissolution lying between pH 4.5 and pH 8.5, the pH of the composition before application being predetermined such that it is further away from pH 7.4 than the dissolution pH of the polymer which constitutes the microparticles, thereby not permitting dissolution of the microparticles before application.

2. An ophthalmic composition according to Claim 1, characterized in that the carboxylic polymer is chosen from cellulose acetate phthalate and a copolymer of methacrylic acid and methyl methacrylate.

3. An ophthalmic composition according to Claim 1 or Claim 2, characterized in that the polymeric microparticles have a particle size of between 20 and 70 μm.

4. An ophthalmic composition according to Claim 3, characterized in that the polymeric microparticles have a particle size of between 32 and 56 μm.

5. An ophthalmic composition according to any one of the preceding Claims, characterized in that the suspension medium is based on polysaccharides or natural or synthetic polysaccharide derivatives.

6. An ophthalmic composition according to any one of the preceding Claims, characterized in that the suspension medium is a gellan gum.

7. An ophthalmic composition according to any one of the preceding Claims, characterized in that the suspension medium contains at least one active principle.

8. An ophthalmic composition according to Claim 7, characterized in that the active principle is identical in the suspension medium and the microparticles.

9. An ophthalmic composition according to any one of the preceding Claims, characterized in that the active principle is chosen from antiglaucoma agents, antibiotics and antiviral agents.

10. An ophthalmic composition according to Claim 9, characterized in that the active principle is timolol.

11. An ophthalmic composition according to any one of the preceding Claims, characterized in that it contains other components chosen from tonicity regulators and preservatives.

12. An ophthalmic composition according to any one of the preceding Claims, characterized in that it is presented in the form of a liquid capable of being applied in drop form.

13. A process for preparing an ophthalmic composition according to Claim 1, characterized in that:
    - the polymer forming the microparticles and the active principle are solubilized in a solvent,
    - the solvent is then evaporated off and the polymer is formed into microparticles, and
    - the microparticles obtained above are then mixed into a non-solid medium compatible with ophthalmic application and optionally containing the other ingredients, until a suspension is obtained.

**Claims for the following Contracting State : ES**

1. A process for preparing a non-solid ophthalmic composition, characterized in that said composition contains a suspension of polymeric microparticles in a non-solid medium compatible with ophthalmic application, said polymeric microparticles being formed from a polycarboxylic polymer or a polycationic polymer and having a pH of dissolution lying between pH 4.5 and pH 8.5, the pH of the composition before application being predetermined such that it is further away from pH 7.4 than the dissolution pH of the polymer which constitutes the microparticles, thereby not permitting dissolution of the microparticles before application; which process comprises the following steps:
    - the polymer forming the microparticles and the active principle are solubilized in a solvent,
    - the solvent is then evaporated off and the polymer is formed into microparticles, and

- the microparticles obtained above are then mixed into a non-solid medium compatible with ophthalmic application and optionally containing the other ingredients, until a suspension is obtained.

2. A process according to Claim 1, characterized in that the carboxylic polymer is chosen from cellulose acetate phthalate and a copolymer of methacrylic acid and methyl methacrylate.

3. A process according to Claim 1 or Claim 2, characterized in that the polymeric microparticles have a particle size of between 20 and 70 µm.

4. A process according to Claim 3, characterized in that the polymeric microparticles have a particle size of between 32 and 56 µm.

5. A process according to any one of the preceding Claims, characterized in that the suspension medium is based on polysaccharides or natural or synthetic polysaccharide derivatives.

6. A process according to any one of the preceding Claims, characterized in that the suspension medium is a gellan gum.

7. A process according to any one of the preceding Claims, characterized in that the suspension medium contains at least one active principle.

8. A process according to Claim 7, characterized in that the active principle is identical in the suspension medium and the microparticles.

9. A process according to any one of the preceding Claims, characterized in that the active principle is chosen from antiglaucoma agents, antibiotics and antiviral agents.

10. A process according to Claim 9, characterized in that the active principle is timolol.

11. A process according to any one of the preceding Claims, characterized in that the other ingredients present in the non-solid medium are chosen from tonicity regulators and preservatives.

## Patentansprüche

## Patentansprüche für folgende Vertragsstaaten : AT, BE, CH, DE, DK, FR, GB, IT, LU, NL, SE

1. Nichtfestes ophthalmisches Präparat, dadurch gekennzeichnet, daß es eine Suspension aus polymeren Mikroteilchen in einem nichtfestem Medium enthält, das mit einer ophthalmischen Anwendung kompatibel ist, wobei die genannten polymeren Mikroteilchen aus einem Polycarbonsäure-Polymeren oder einem polykationischen Polymeren gebildet werden und einen pH-Wert zum Auflösen aufweisen, der zwischen pH 4,5 und pH 8,5 liegt, wobei der pH-Wert des Präparates vor der Anwendung so bestimmt wird, daß er weiter entfernt von pH 7,4 ist als der pH zum Auflösen des Polymeren, das die Mikroteilchen bildet, wodurch das Auflösen der Mikroteilchen nicht vor dem Anwenden ermöglicht wird.

2. Ophthalmisches Präparat nach Anspruch 1, dadurch gekennzeichnet, daß das Carbonsäure-Polymere aus Celluloseacetatphthalat und einem Copolymeren aus Methacrylsäure und Methylmethacrylat ausgewählt wird.

3. Ophthalmisches Präparat nach Anspruch 1 oder Anspruch 2, dadurch gekennzeichnet, daß die polymeren Mikroteilchen eine Teilchengröße zwischen 20 und 70 µm aufweisen.

4. Ophthalmisches Präparat nach Anspruch 3, dadurch gekennzeichnet, daß die polymeren Mikroteilchen eine Teilchengröße zwischen 32 und 56 µm aufweisen.

5. Ophthalmisches Präparat nach einem der vorgenannten Ansprüche, dadurch gekennzeichnet, daß das Suspensions-medium Polysaccharide oder natürliche oder synthetische Polysaccharid-Derivate als Grundlage aufweist.

6. Ophthalmisches Präparat nach einem der vorgenannten Ansprüche, dadurch gekennzeichnet, daß das Suspensionsmedium ein Geliergummi ist.

7. Ophthalmisches Präparat nach einem der vorgenannten Ansprüche, dadurch gekennzeichnet, daß das Suspensionsmedium mindestens ein Wirkprinzip enthält.

8. Ophthalmisches Präparat nach Anspruch 7, dadurch gekennzeichnet, daß das Wirkprinzip in Suspensionsmedium und Mikroteilchen identisch ist.

9. Ophthalmisches Präparat nach einem der vorgenannten Ansprüche, dadurch gekennzeichnet, daß das Wirkprinzip ausgewählt wird aus Antiglaucom-Mitteln, Antibiotika und antiviralen Mitteln.

10. Ophthalmisches Präparat nach Anspruch 9, dadurch gekennzeichnet, daß das Wirkprinzip Timolol ist.

11. Ophthalmisches Präparat nach einem der vorgenannten Ansprüche, dadurch gekennzeichnet, daß es weitere Komponenten enthält, die aus Tonusregulatoren und Konservierungsstoffen ausgewählt werden.

12. Ophthalmisches Präparat nach einem der vorgenannten Ansprüche, dadurch gekennzeichnet, daß es in Form einer Flüssigkeit vorliegt, die in Tropfenform angewendet werden kann.

13. Verfahren zur Herstellung eines ophthalmischen Präparats nach Anspruch 1, das dadurch gekennzeichnet ist, daß
   - das Polymere, das die Mikroteilchen bildet, und das Wirkprinzip in einem Lösungsmittel gelöst werden,
   - das Lösungsmittel dann eingedampft und das Polymere zu Mikroteilchen geformt wird und
   - die oben erhaltenen Mikroteilchen dann in ein nichtfestes Medium eingemischt werden, das mit der Anwendung am Auge kompatibel ist und gegebenenfalls die anderen Bestandteile enthält, bis eine Suspension erhalten wird.

**Patentansprüche für folgenden Vertragsstaat : ES**

1. Verfahren zur Herstellung eines nichtfesten ophthalmischen Präparats, dadurch gekennzeichnet, daß das genannte Präparat eine Suspension aus polymeren Mikroteilchen in einem nichtfestem Medium enthält, das mit einer ophthalmischen Anwendung kompatibel ist, wobei die genannten polymeren Mikroteilchen aus einem Polycarbonsäure-Polymeren oder einem polykationischen Polymeren gebildet werden und einen pH-Wert zum Auflösen aufweisen, der zwischen pH 4,5 und pH 8,5 liegt, wobei der pH-Wert des Präparates vor der Anwendung so bestimmt wird, daß er weiter entfernt von pH 7,4 ist als der pH zum Auflösen des Polymeren, das die Mikroteilchen bildet, wodurch das Auflösen der Mikroteilchen nicht vor dem Anwenden ermöglicht wird, wobei das Verfahren die folgenden Stufen umfaßt:
   - das Polymere, das die Mikroteilchen bildet, und das Wirkprinzip werden in einem Lösungsmittel gelöst,
   - das Lösungsmittel wird dann eingedampft, und das Polymere wird zu Mikroteilchen geformt; und
   - die oben erhaltenen Mikroteilchen werden dann in ein nichtfestes Medium eingemischt, das mit der Anwendung am Auge kompatibel ist und gegebenenfalls die anderen Bestandteile enthält, bis eine Suspension erhalten wird.

2. Verfahren nach Anspruch 1, dadurch gekennzeichnet, daß das Carbonsäure-Polymere aus Celluloseacetatphthalat und einem Copolymeren aus Methacrylsäure und Methylmethacrylat ausgewählt wird.

3. Verfahren nach Anspruch 1 oder Anspruch 2, dadurch gekennzeichnet, daß die polymeren Mikroteilchen eine Teilchengröße zwischen 20 und 70 μm aufweisen.

4. Verfahren nach Anspruch 3, dadurch gekennzeichnet, daß die polymeren Mikroteilchen eine Teilchengröße zwischen 32 und 56 μm aufweisen.

5. Verfahren nach einem der vorgenannten Ansprüche, dadurch gekennzeichnet, daß das Suspensionsmedium Polysaccharide oder natürliche oder synthetische Polysaccharid-Derivate als Grundlage aufweist.

6. Verfahren nach einem der vorgenannten Ansprüche, dadurch gekennzeichnet, daß das Suspensionsmedium ein Geliergummi ist.

7.  Verfahren nach einem der vorgenannten Ansprüche, dadurch gekennzeichnet, daß das Suspensionsmedium mindestens ein Wirkprinzip enthält.

8.  Verfahren nach Anspruch 7, dadurch gekennzeichnet, daß das Wirkprinzip in Suspensionsmedium und Mikroteilchen identisch ist.

9.  Verfahren nach einem der vorgenannten Ansprüche, dadurch gekennzeichnet, daß das Wirkprinzip ausgewählt wird aus Antiglaucom-Mitteln, Antibiotika und antiviralen Mitteln.

10.  Verfahren nach Anspruch 9, dadurch gekennzeichnet, daß das Wirkprinzip Timolol ist.

11.  Verfahren nach einem der vorgenannten Ansprüche, dadurch gekennzeichnet, daß die weiteren, in dem nichtfesten Medium vorhandenen Komponenten aus Tonusregulatoren und Konservierungsstoffen ausgewählt werden.


## Revendications

**Revendications pour les Etats contractans suivants : AT, BE, CH, DE, DK, FR, GB, IT, LU, NL, SE**

1.  Composition ophtalmique non solide caractérisée en ce qu'elle comporte une suspension de microparticules polymériques dans un milieu non solide compatible avec l'application ophtalmique, lesdites microparticules polymériques solides étant formées à partir d'un polymère polycarboxylique ou d'un polymère polycationique et ayant un pH de dissolution situé entre environ un pH de 4,5 et un pH de 8,5, le pH de la composition avant l'application étant prédéterminé de telle sorte qu'il soit plus éloigné du pH 7,4 que ne l'est le pH de dissolution du polymère qui constitue les microparticules, ne permettant pas de ce fait la dissolution des microparticules avant l'application.

2.  Composition ophtalmique selon la revendication 1, caractérisée en ce que le polymère carboxylique est choisi parmi l'acétophtalate de cellulose et un copolymère d'acide méthacrylique et de méthacrylate de méthyle.

3.  Composition ophtalmique selon la revendication 1 ou 2, caractérisée en ce que les microparticules polymériques ont une taille de particules entre 20 et 70 µm.

4.  Composition ophtalmique selon la revendication 3, caractérisée en ce que les microparticules polymériques ont une taille de particules entre 32 et 56 µm.

5.  Composition ophtalmique selon l'une quelconque des revendications précédentes, caractérisée en ce que le milieu de suspension est à base de polysaccharides ou de dérivés de polysaccharides naturels ou synthétiques.

6.  Composition ophtalmique selon l'une quelconque des revendications précédentes, caractérisée en ce que le milieu de suspension est un gellan gum.

7.  Composition ophtalmique selon l'une quelconque des revendications précédentes, caractérisée en ce que le milieu de suspension contient au moins un principe actif.

8.  Composition ophtalmique selon la revendication 7, caractérisée en ce que le principe actif est identique dans le milieu de suspension et dans les microparticules.

9.  Composition ophtalmique selon l'une quelconque des revendications précédentes, caractérisée en ce que principe actif est choisi parmi des agents anti-glaucomes, des antibiotiques et des agents antiviraux.

10.  Composition ophtalmique selon la revendication 9, caractérisée en ce que le principe actif est du timolol.

11.  Composition ophtalmique selon l'une quelconque des revendications précédentes, caractérisée en ce qu'elle contient d'autres composants choisis parmi des régulateurs de tonicité et des conservateurs.

12. Composition ophtalmique selon l'une quelconque des revendications précédentes, caractérisée en ce qu'elle est présentée sous forme liquide capable d'être appliquée sous forme de goutte.

13. Procédé pour préparer une composition ophtalmique selon la revendication 1, caractérisé en ce que :
    - le polymère constituant les microparticules et le principe actif sont solubilisés dans un solvant,
    - le solvant est ensuite évaporé et le polymère mis sous forme de microparticules, et
    - les microparticules obtenues précédemment sont ensuite mélangées dans un milieu non solide compatible avec l'application ophtalmique et contenant éventuellement les autres ingrédients, jusqu'à l'obtention d'une suspension.

**Revendications pour l'Etat contractant suivant : ES**

1. Procédé pour préparer les compositions ophtalmiques non solides, caractérisé en ce que ladite composition contient une suspension de microparticules polymériques dans un milieu non solide compatible avec une application ophtalmique, lesdites microparticules polymériques étant formées à partir d'un polymère carboxylique ou d'un polymère polycationique et ayant un pH de dissolution situé entre environ un pH de 4,5 et un pH de 8,5, le pH de la composition avant l'application étant prédéterminé de telle sorte qu'il soit plus éloigné du pH 7, 4 que ne l'est le pH de dissolution du polymère qui constitue les microparticules, ne permettant pas de ce fait la dissolution des microparticules avant l'application; lequel procédé comprend les étapes suivantes :
    - le polymère constituant les microparticules et le principe actif sont solubilisés dans un solvant,
    - le solvant est alors évaporé et le polymère mis sous forme de microparticules, et
    - les microparticules obtenues précédemment sont ensuite mélangées dans un milieu non solide compatible avec l'application ophtalmique et contenant éventuellement les autres ingrédients, jusqu'à l'obtention d'une suspension.

2. Procédé selon la revendication 1, caractérisé en ce que le polymère carboxylique est choisi parmi l'acétophtalate de cellulose et un copolymère d'acide méthacrylique et de méthacrylate de méthyle.

3. Procédé selon la revendication 1 ou 2, caractérisé en ce que les microparticules polymériques ont une taille de particules entre 20 et 70 $\mu$m.

4. Procédé selon la revendication 3, caractérisé en ce que les microparticules polymériques ont une taille de particules entre 32 et 56 $\mu$m.

5. Procédé selon l'une quelconque des revendications précédentes, caractérisé en ce que le milieu de suspension est à base de polysaccharides ou de dérivés de polysaccharides naturels ou synthétiques.

6. Procédé selon l'une quelconque des revendications précédentes, caractérisé en ce que le milieu de suspension est un gellan gum.

7. Procédé selon l'une quelconque des revendications précédentes, caractérisé en ce que le milieu de suspension contient au moins un principe actif.

8. Procédé selon la revendication 7, caractérisé en ce que le principe actif est identique dans le milieu de suspension et dans les microparticules.

9. Procédé selon l'une quelconque des revendications précédentes, caractérisé en ce que le principe actif est choisi parmi des agents anti-glaucomes, des antibiotiques et des agents antiviraux.

10. Procédé selon la revendication 9, caractérisé en ce que le principe actif est du timolol.

11. Procédé selon l'une quelconque des revendications précédentes, caractérisé en ce que les autres ingrédients présents dans le milieu non solide sont choisis parmi les régulateurs de tonicité et les conservateurs.